# EUROPEAN PATENT APPLICATION

(11) **EP 4 765 142 A1**
(43) Date of publication of application: **24.06.2026**
(21) Application number: 24223069.6
(22) Date of filing: 23.12.2024
(51) Int. Cl.: G16H 20/40, G16H 50/20

(54) **IMPROVED PREOPERATIVE MEDICINE INVOLVING SEMANTIC AI**

(71) Applicant: Van Overmeire, Henri, 9052 Gent (BE)
(72) Inventor: Van Overmeire, Henri, 9052 Gent (BE)
(74) Representative: Patentales BV

(57) **Abstract**

The invention provides, amongst other aspects, a device comprising: an input interface; a control module connected to the input interface; an output interface connected to said control module; wherein the control module is configured, upon initiation of a respective next measurement cycle, to: receive, via the input interface, respective next measurement data measured with respect to a patient; process the respective next measurement data, based at least on a respective previous graph associated with a respective previous measurement cycle, for obtaining a respective next graph associated with the respective next measurement cycle; output, via the output interface, the respective next graph; wherein the previous graph and the next graph each relate to knowledge graphs and each comprise: a plurality of entities relevant to a clinical scenario to which the respective measurement cycles relate, and a plurality of relations between the entities; and wherein the previous and the next graph differ in at least one entity or relation.

## Description

### Field of the invention

The present invention relates to improved perioperative medicine through a comprehensive and computational semantic framework. Particularly, the invention relates to the application of knowledge graphs in a clinical context.

### Background art

The management of perioperative and critical care medicine increasingly relies on advanced technologies to optimize patient outcomes. The complexity of medical device integration, particularly in high-stakes environments like operating rooms and intensive care units, necessitates systems capable of interpreting vast amounts of heterogeneous data in real-time.

Current solutions often fall short in providing cohesive and adaptive decision support due to fragmented data streams and limited contextual integration. Advancements in artificial intelligence (AI) and semantic data processing have created opportunities to enhance device control and clinical decision-making.

A notable approach is the use of semantic knowledge graphs, which enable the synthesis of real-time data streams with contextual information derived from patient health records, procedural data, and physiological models. These graphs provide a structured, interpretable representation of clinical scenarios, facilitating automated reasoning and decision-making. Despite these advancements, integrating diverse data sources, ensuring real-time adaptability, and maintaining system explainability remain significant challenges.

Existing systems often lack the capacity to holistically consider the causative relationships between physiological changes and device settings, limiting their effectiveness in dynamic clinical environments.

US20230253117A1 discloses a method for determining an assessment of a patient for a medical condition. This involves a knowledge graph that is computed based on the input medical data. An issue with US20230253117A1 remains the lack of explainability. US20230253117A1 relies on a knowledge graph to generate a vector representing a state of the patient. This vector is then carried over to a next step, and an assessment of the patient for a medical condition is determined using a machine learning based network based on that vector. The approach of US20230253117A1 is thus directed to vector representation and not to a model that is explainable to an operator (medical staff).

CN117316465A discloses a method for constructing a multi-mode knowledge graph for nursing. However, CN117316465A is directed to providing feedback to the user on corresponding nursing knowledge based on a multimodal knowledge graph based on a nursing query instruction entered by the user, and not to real-time measurements. The approach of CN1 17316465A therefor lacks effectiveness in dynamic clinical environments.

The present invention aims at addressing issues, such as the issues mentioned above.

### Summary of the invention

According to a first aspect, the present invention provides a device comprising:
an input interface;
a control module connected to the input interface;
an output interface connected to said control module;
wherein the control module is configured, upon initiation of a respective next measurement cycle, to:
   receive, via the input interface, respective next measurement data measured with respect to a patient;
   process the respective next measurement data, based at least on a respective previous graph associated with a respective previous measurement cycle, for obtaining a respective next graph associated with the respective next measurement cycle;
   output, via the output interface, the respective next graph;
   wherein the previous graph and the next graph each relate to knowledge graphs and each comprise: a plurality of entities relevant to a clinical scenario to which the respective measurement cycles relate, and a plurality of relations between the entities; and
   wherein the previous and the next graph differ in at least one entity or relation.

Thereby, the measurement data may relate to one or more input streams (with, e.g., a waveform per input stream), and the processing may be referred to as semantic stream processing.

Through these features, the invention may advantageously combine semantic artificial intelligence techniques with graph-based data structures and real-time signal processing. The invention may thereby enable precise control of medical devices, such as mechanical ventilators, by dynamically adapting to patient-specific and clinical-scenario-aware contexts, thus addressing critical gaps in current technologies. Importantly, such advantages may be provided in a way that is explainable to the operator (medical staff), in view of the temporal sequence of graphs.

These advantages are not provided by US20230253117A1, relies on a knowledge graph to generate a vector representing a state of the patient. This vector is then carried over to a next step, and an assessment of the patient for a medical condition is determined using a machine learning based network based on that vector. The approach of US20230253117A1 is directed to a vector representation that is not "explainable", as the knowledge graph remains static. Any update of the knowledge graph does not relate to any "online" update but instead relates to an "offline" updating of the pre-determined graph. Hence, the knowledge graph of US20230253117A1 is to be construed as a static single graph that lacks any form of dynamic features. US20230253117A1 does describe some basic display of information according to "an assessment", e.g., "a risk or severity score representing the risk or severity of the medical condition for the patient", or a "likelihood of ventilator need", see par. [0036], but this merely relates to the assessment by itself, and does not allow for any explainability of how the assessment is arrived at.

Also, these advantages are not provided by CN117316465A. CN117316465A is directed to providing feedback based on queries entered by the user, which is not adapted to the needs of dynamic clinical environments.

In embodiments, the processing involves natural language processing (NPL).

In embodiments, the device comprises a postprocessing module relating to a large language model (LLM), with at least one graph, preferably the next graph, as input prompt; whereby the LLM output is displayed to the operator, either along with the at least one graph, or instead of the at least one graph. Such embodiments advantageously provide for enhanced explainability, through natural language output. This may allow the operator to respond faster or more adequately to unexpected changes in a patient's condition and may thus allow for better patient care.

According to a second aspect, the invention provides a system comprising:
a device, the device comprising
   an input interface;
   a control module connected to the input interface; an output interface connected to said control module;
a data source module, preferably a sensor module, for connection to the input interface;
a user module comprising at least a screen for display to an operator and for connection to the output interface;
wherein the control module is configured to:
   receive, from the data source module, respective next measurement data; process the respective next measurement data, based at least on a respective previous graph associated with a respective previous measurement cycle, for obtaining a respective next graph associated with the respective next measurement cycle;
   send data of the respective next graph to the user module;
wherein the data source module is configured to:
   measure next measurement data with respect to a patient, and send the next measurement data to the input interface of the device;
wherein the user module is configured to:
   receive, from the output interface of the device, data of at least one graph;
   display, on said screen, said at least one graph;
wherein the previous graph and the next graph each relate to knowledge graphs and each comprise:
   a plurality of entities relevant to a clinical scenario to which the respective measurement cycles relate, and
   a plurality of relations between the entities; and
wherein the previous and the next graph differ in at least one entity or relation.

According to a further aspect, the invention provides a method for supporting a clinical event according to a clinical scenario, comprising the steps of:
receiving, via an input interface comprised in a device, respective next measurement data measured with respect to a patient;
processing, by a control module comprised in the device the respective next measurement data based at least on a respective previous graph associated with a respective previous measurement cycle, for obtaining a respective next graph associated with the respective next measurement cycle;
outputting, via an output interface comprised in the device, the respective next graph;
wherein the previous graph and the next graph each relate to knowledge graphs and each comprise:
   a plurality of entities relevant to a clinical scenario to which the respective measurement cycles relate, and
   a plurality of relations between the entities; and
wherein the previous and the next graph differ in at least one entity or relation.

In embodiments, the method further comprises:
sending, based on the difference between the graph associated with the previous measurement cycle and the graph associated with the new measurement cycle, an instruction to the patient-related device for altering an operational parameter of the patient-related device.

In embodiments, the method further comprises:
displaying, via a screen comprised in a user module connected to the output interface of the device, at least one graph, said at least one graph relating to at least one of the previous graph and the next graph.

Preferred embodiments and their advantages are provided in the description and the dependent claims.

### Brief description of the drawings

The present invention will be discussed in more detail below, with reference to the attached drawings.
**Fig. 1** illustrates an example system according to the invention.
**Fig. 2** illustrates an example application of the system according to the invention.
**Fig. 3** shows an example of a pre-determined patient-specific graph according to the invention.
**Fig. 4** shows an example next graph according to the invention.

### Description of embodiments

The following descriptions depict only example embodiments and are not considered limiting in scope. Any reference herein to the disclosure is not intended to restrict or limit the disclosure to exact features of any one or more of the exemplary embodiments disclosed in the present specification.

Furthermore, the terms first, second, third and the like in the description and in the claims are used for distinguishing between similar elements and not necessarily for describing a sequential or chronological order. The terms are interchangeable under appropriate circumstances and the embodiments of the invention can operate in other sequences than described or illustrated herein.

Furthermore, the various embodiments, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present invention, the only enumerated components of the device are A and B, and further the claim should be interpreted as including equivalents of those components.

In this document, the term "knowledge graph" refers to a structured representation of knowledge that organizes information as a network of entities (nodes) and their relationships (edges). It is used to model real-world concepts and their interconnections in a way that is both human-readable and machine-interpretable. In embodiments, entities relate to any or any combination of objects, concepts, or instances, such as people, places, medical measurements, or devices. Relationships represent meaningful connections or associations between entities, such as "causes," "measured by," or "belongs to." Entities and relationships can have attributes that describe properties or values, such as "has a certain clinical condition," "time," or "status." In the context of the invention, the structure of the graph captures how entities relate to one another for a specific clinical scenario. In example embodiments, the next graph and/or the pre-determined graph include nodes for one or more of: arterial blood pressure, hypotension, ventilation flow, and mechanical ventilator, with edges describing relationships like "affects," "measured by," or "suspect cause." The temporal sequence of next graphs may thereby allow to identify changes in these nodes and their relationships, allowing to identify clinical trends, issue alerts, or adjust medical device settings dynamically, as described in this document.

Advantages of the invention are described in the summary section. Particularly advantageous may be the provision, through the generation of graphs, of a comprehensive, modular, multi-domain ontological framework. It may allow tailoring to the clinical reality of perioperative medicine and incorporation into an explainable and personalized recommendation engine. Related, it may allow its performance to be objectively evaluated and may serve as a decision engine for further integrations.

In embodiments, the input side of the device relates to two kinds of data: numeric (real-time) streaming data originating from any kind of medical device used in perioperative medicine. Numerical data can be delivered to the system in the form of waveforms (high sampling frequency), or as pre-processed inputs (e.g., averaged values, min/max etc). This distinction may be important given the fact that physiological signals are typically measured in a variety of ways, leading to different kinds of signals. The signal of an invasive blood pressure catheter (intra-arterial line) is typically a pseudo-continuous signal, whereas information regarding arterial blood pressure from oscillometric devices is typically presented as discrete measurement points with a lower sampling frequency (e.g., 1/60 Hz). In preferred embodiments, the device is capable of handling measurement data of both kinds. In principle, any kind of numerical monitoring data may thereby serve as an input to the device. The stream processing architecture may include ventilator settings. It may additionally, or alternatively, incorporate monitor waveforms, one or more, including but not limited to the measurement of arterial blood pressure, the measurement of central venous pressure, and the pressure aspects of the mechanical ventilation cycle. In embodiments, peritoneal insufflation settings are supported with monitor waveforms, including measurements of arterial blood pressure, central venous pressure, and mechanical ventilation pressure aspects. In embodiments, procedural information is another source, with monitor waveforms that include physical positioning of the patient, central venous pressure measurement, and mechanical ventilation cycle pressures. In embodiments, the measurement data and/or a pre-determined graph comprises contextual information regarding the patient and/or about the operative procedure (or, equivalently, clinical scenario), and/or a mechanistic schema that bridges the incorporation of numerical streaming data into a labelled-property graph model. This knowledge graph may be instantiated from a custom ontology, with the help of large language models and graph-native algorithms as well as machine learning procedures. In embodiments, based on measurement data and optionally also a pre-determined graph, the system can estimate a suitable input for clinical targets such as blood pressure, ventilation rate, and acid-base status. In preferred embodiments, the operator (health care physician) has the possibility to specify or overrule preset targets.

In embodiments, the user input means relates to an LLM (large language model). This may provide for user-friendly interfacing with the invention.

In embodiments, the invention is directed at control of a patient-related device without any display being available for an operator. Such embodiments may still enjoy the benefits known for the use of knowledge graphs, since the modus operandi of such may still be understood from the graph present in system logs. In embodiments, the invention is at the display of a graph being available for an operator, without any control of a patient-related device. Such embodiments may equally enjoy the benefits known for the use of knowledge graphs, effectively assisting an operator through automated interpretation of a temporal sequence of measurement data.

In embodiments, the processing of measurement data, or, equivalently, the semantic stream processing, relates to the following processing of input streams. First, each input stream (e.g., waveform) may be converted into a temporal sequence of knowledge graphs that is derived from a custom ontology. The graph nodes represent waveform features and clinical concepts (e.g., hypotension) in various window settings (short-term, medium-term, long-term). Translating the input waveforms into a sequence of labelled-property graphs corresponds to the way human healthcare operators process changes in the waveforms: over the long term, the patient has been hemodynamically stable, over the short term, there is a major drop in blood pressure, signifying an acute problem. Additional annotations to the input streams can be made for further processing by an inference engine (e.g., regarding data quality).

In embodiments, a means for causality assessment may be provided. This may relate, e.g., to combining computed results from graph-native reasoning **and/or** graph-based feature extraction of the mentioned input (e.g., waveforms and pre-determined (contextual) graph) **and/or** a fuzzy logic inference engine **and/or** a causal effect variational auto-encoder neural network. Such means may allow an assessment to be made regarding the strength of the causal relation between the physiological changes and current output device settings. Such strength may advantageously be displayed as annotation to a displayed graph, and/or be shown directly to the operator and/or form the basis for a modification of a control of a patient-related device.

In embodiments, a means for effect estimation may be provided. Through the causative model, the effects of individual and combined changes in actuator settings may be estimated, preferably not only on the target variables but also on underlying physiological variables. This may enable to reason within a broader context. For instance, increasing vasopressor dosage (actuator) in a patient with underlying vascular disease of the splanchnic vessels (context) may lead to increased risk of intestinal ischemia (physiological concept) despite increased blood pressure (output variable). By instantiating the knowledge graph further around the concept ('intestinal ischemia'), the invention may enable to discern that lactate tests are of value. If it is detected that this information is already presented, the accompanying semantic stream may be interpreted for this parameter; this allow to take this into account in the final inference of the course of action.

In embodiments, a means for enhanced language output is provided. This may enable the invention to converse clearly about its internal deductive and inductive reasoning processes with operators (health care staff), in any form possible (written, speech). Thereby, in preferred embodiments, the graphs (next graphs, pre-determined (contextual) graph (if any)) may serve as a basis for textual output generated by a large language model. Several approaches are possible: prompt engineering (including contextual information in the prompt input for a large language model), or incorporating this contextual information in the training data for the finetuning of a large language model. A suggested course of action may thereby be presented. Additionally, or alternatively, an altering of an operational parameter of a patient-related device may be provided.

In embodiments, means are provided for inferring (more) optimal output device settings. This may relate to the input graphs (measurement data) and final ("causally enriched") contextual graphs to serve as complementary inputs to a prediction head engine that outputs a suggested output state configuration for any output device present. Several graph algorithms can be employed to derive features, either local or topological, that serve as an input to the prediction head. In embodiments, the prediction head engine relates to any or any combination of: fuzzy logic regression inference, shallow or deep learning models.

In embodiments, the processing of measurement data, or, equivalently, the semantic stream processing, relates to a real-time data architecture wherein measurement data is fed to a streaming pipeline (block 1). Block 1 exchanges information regarding stream enrichment/transformation with a stream processor (block 2). Block 1 feeds its output to a real-time analytics agent (block 3). Block 3 feeds its output to a real-time analytics visualisation/decisioning agent (block 4). Optionally, block 4 is enhanced with monitoring/alert functionality, which receives direct input from block 2 to ensure swift handling of urgencies. In related embodiments, the control unit comprises a Streaming Pipeline which functions as a general information carrying layer. The streaming pipeline may allow the device to handle unbounded streams, given that adequate procedural usage of a generic stream processing component (e.g., Apache Flink) with a semantic engine (e.g., as provided by Neo4j Graph Database system with the Neo4j Semantics plugin) allows for the mentioned transformation into a sequence of semantic graph structures.

In preferred embodiments, the invention involves the use of a custom ontological organizing principle and stream processing subsystems. Preferably, the input data streams are then converted into a sequence of knowledge graphs that represent the clinically relevant information contained in their signal content. For example, the sequence of graphs may form a continuous situational assessment of the input waveforms.

In preferred embodiments, the device internally represents that which can be contextual input, in the form of a knowledge graph. This may allow for improved integration in a clinical context. Thereby, this graph may be instantiated guided by a variety of organizing principles, e.g., a (basic) taxonomy or a complicated ontology that represents causal or relational information on the entities relevant to the clinical scenario and physiological principles underpinning the reasoning principles that will be employed in the inference stage. In related embodiments, an ontology can be implemented using standard techniques, e.g.: Web Ontology Language (OWL).

For correct instantiation of the contextual knowledge graph, in preferred embodiments, the device makes use of one or more than one of a variety of input sources, comprising textual (reports) and numerical (e.g.: lab values or clinical scoring systems) information from the Electronic Health Records and external sources (e.g.: existing subontologies such as SNOMED-CT).

In embodiments, instantiation of the pre-determined knowledge graph relies on LLMs to match patient-related input to the structure of the graph.

In embodiments, obtaining the next graph relies on LLMs to incorporate input from the operator provided via the user input means, wherein the user input is used as input prompt and the LLM output is used for obtaining the next graph.

In preferred embodiments, the graphs of the invention relate to an overarching clinical context that is captured in a dedicated causal knowledge graph, which integrates information concerning the individual patient characteristics, underlying pathophysiological changes, specific aspects of a surgical procedure (if any) and mechanistic physiological models.

In embodiments, the patient-related device is at least partially controlled by the device of the invention. In related embodiments, the patient-related device partially controlled by the device generates measurement data which feeds, via the input interface, into the device. In yet other embodiments, at least part of the measurement data originates from a patient-related device which is not controlled in any way by the device of the invention.

In embodiments, the user input means relates to one or more than one of the following: touchscreen interfaces; mouse or trackpad devices; styluses or digital pens; voice input systems; joysticks or control knobs; graphical buttons and sliders on-screen; gesture recognition systems that track, e.g., hand or body movements; haptic feedback devices; biometric input systems.

In embodiments, the previous measurement cycle and the next measurement cycle belong to a temporal sequence of at least five respective cycles, preferably at least ten cycles, and wherein a maximum time between respective cycles is less than one minute, preferably less than 30 seconds, more preferably less than 10 seconds. This may advantageously provide real-time or near-real-time operation.

In embodiments, the output interface comprises electronic connection means for controlling a patient-related device, and wherein the control module is further configured to: send, based on the difference between the graph associated with the previous measurement cycle and the graph associated with the new measurement cycle, an instruction to the patient-related device for altering an operational parameter of the patient-related device. In embodiments, the instruction thereby triggers an altering-related action. The altering-related action may relate to altering said operational parameter. Additionally, or alternatively, the altering-related action may relate to generating a patient-related device alert via an alerting means comprised in the patient-related device. For instance, the instruction may trigger the patient-related device to emit an acoustic alert. This may have the advantage that an operator, intuitively and directly, receives the alert at a point where an issue may be detected.

In embodiments, the patient-related device relates to a mechanical ventilator, wherein the operational parameter relates at least to an intensity of ventilation flow, and wherein the respective next measurement data relate to at least one of, preferably at least two of: arterial blood pressure, central venous pressure, oxygen saturation in the blood, end-tidal carbon dioxide content, a pressure value characteristic of the ventilator, a volume value characteristic of the ventilator.

In embodiments, the patient-related device relates to a peritoneal insufflator, wherein the operational parameter relates at least to an intensity of insufflation, and wherein the respective next measurement data relate to at least one of, preferably at least two of: arterial blood pressure, central venous pressure, a pressure value characteristic of a mechanical ventilation cycle.

In embodiments, the output interface comprises electronic connection means for connecting to a user module comprising a screen for display to an operator, and wherein the control module is further configured to: display, via said screen, at least one graph, said at least one graph relating to at least one of the previous graph and the next graph.

In embodiments, the user module further comprises user input means for receiving input from the operator, and wherein the at least one graph being displayed comprises at least one entity and/or at least one relation that is editable by the operator via said user input means.

In embodiments, the user input means relates to the screen being a touchscreen.

In embodiments, the control module is further configured to: determine, based at least on the next graph, whether an alert for the operator should be generated; if yes, generate said alert and display the alert via said screen.

In embodiments, the alert relates to procedural information and comprises instructions to be carried out by the operator.

In embodiments, the user module further comprises user input means for receiving input from the operator, wherein the alert relates to a prompt for receiving a response from the operator via said user input means, and wherein the control module is further configured to: display, on said screen and along with the prompt and the at least one displayed graph, an annotation of said displayed graph for indicating a predicted impact of the response.

In embodiments, the processing of the next measurement data for obtaining a respective next graph is further based on a pre-determined patient-related graph instantiated based on pre-determined patient data specific to the patient.

In embodiments, the system further comprises: a patient-related device connected to the output interface; wherein the control module is further configured to: send, based on the difference between the graph associated with the previous measurement cycle and the graph associated with the new measurement cycle, an instruction to the patient-related device for altering an operational parameter of the patient-related device; wherein the patient-related device is configured to: receive, from the output interface of the device, the instruction for altering the operational parameter; executing, based on said instruction, an altering-related action; wherein the altering-related action relates to altering said operational parameter and/or generating a patient-related device alert via an alerting means comprised in the patient-related device, e.g., an acoustic alert.

In embodiments, the next graph generated by the device is fed to a postprocessing module.

In embodiments, the postprocessing module relates to an LLM, with the graph as input prompt, whereby the LLM output may be displayed to the operator, either along with one of the graphs, or instead of the graph. In embodiments, the postprocessing relates to a decision support tool. This may advantageously allow enhancing decision-making through the connection of disparate data sources and identification of patterns. In embodiments, the postprocessing relates to AI reasoning. In embodiments, the postprocessing relates to data integration, e.g., involving combining heterogeneous data into a unified, structured format for analysis and/or visualization. In embodiments, the postprocessing relates to search and querying capabilities that are improved through contextual and relational queries enabled by the graph structure.

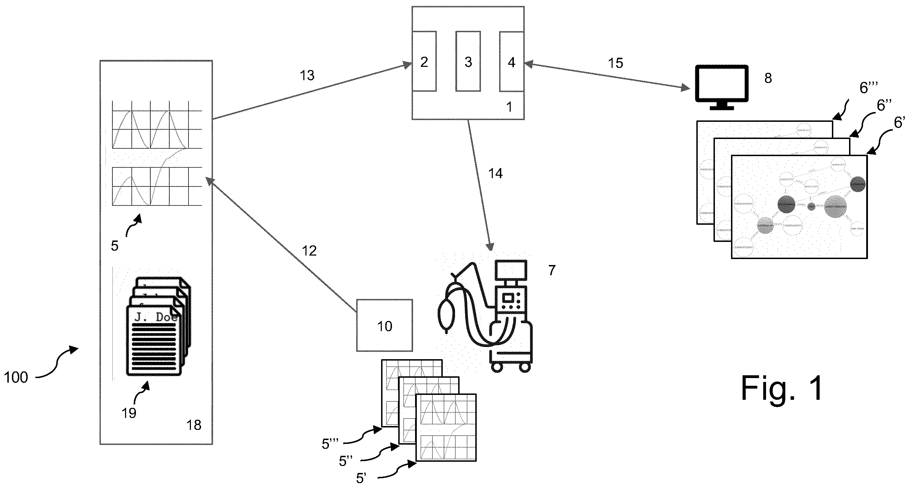

Example embodiments of the invention will be described with reference to Figs. 1-4.

### Example: example system, application and graphs according to the invention

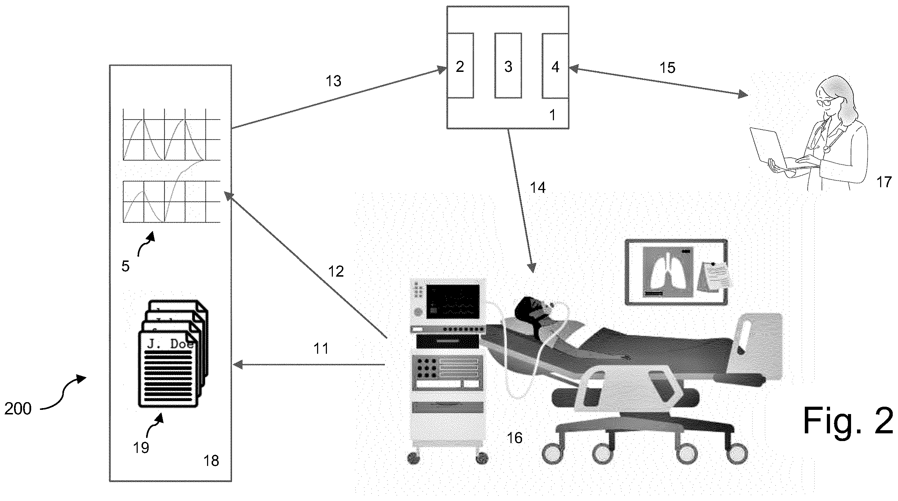

Fig. 1 illustrates an example system 100 according to the invention. It relates to example embodiments where the device 1 belongs to a system 100 that comprises a user module being a screen 8 and a patient-related device being a mechanical ventilator 7. Fig. 2 thereby illustrates an according example application of this example system, with the presence of a patient receiving treatment involving mechanical ventilation, through the mechanical ventilator 7 (shown as integrated with further devices on Fig. 2) and an operator 17 being a medical doctor.

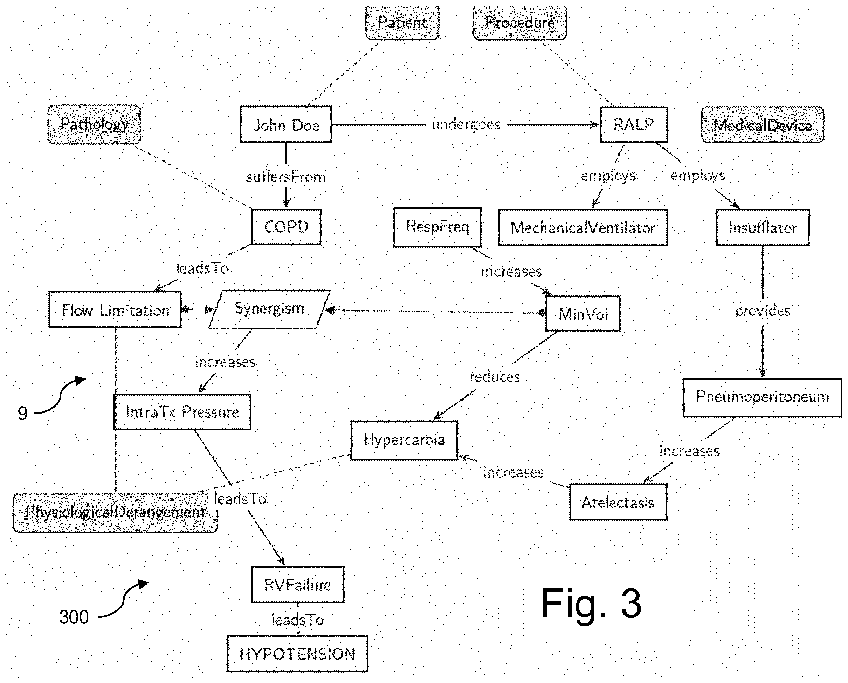

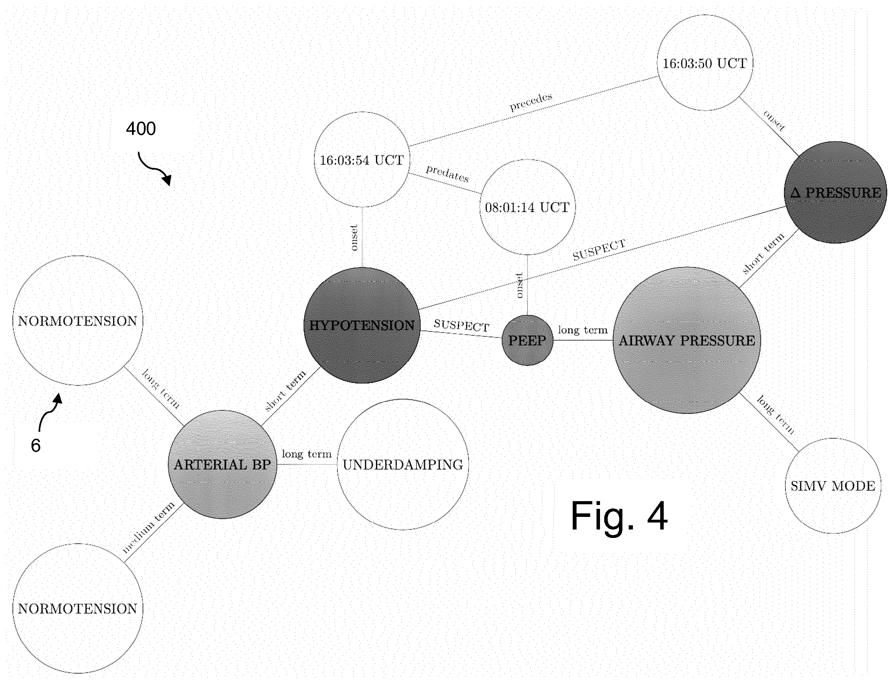

The behaviour of the system 100 is governed by graphs, and updating of graphs (determining of the next graph) based on real-time measurements. Fig. 3 thereby shows an example of a pre-determined patient-specific graph 300 according to the invention. As shown in Fig. 3, the graph is a scenario-specific graph 9 relating to a first example clinical scenario that may involve both a respirator and an insufflator. On the other hand, Fig. 4 shows an example next graph 400 according to the invention, i.e., an intermediate graph that is recomputed for each next measurement cycle.

For illustration purposes, the intermediate graph relates to a scenario-specific next graph 6 relating to a second example clinical scenario, which may be different from the first example clinical scenario. It may be clear that, based on the scenario-specific graph 9 relating to the first example clinical scenario, scenario-specific next graphs (not shown) relating to the first example clinical scenario may be determined. Likewise, correspondence may be established between a scenario-specific graph (not shown) relating to the second example clinical scenario, and the scenario-specific next graphs 6 relating to the second example clinical scenario. Such correspondence is assumed for the system 100 illustrated in Fig. 1 and the system illustrated in Fig. 2, with the next graphs 6', 6" and 6‴ depicted on Fig. 1 and the corresponding pre-determined graph (not shown used for instantiation of the first of these graphs.

Focusing on Fig. 1, the system 100 comprises a device 1. The device 1 comprises an input interface 2, a control module 3 connected to the input interface, and an output interface 4 connected to said control module.

Fig. 1 illustrates a stream of measurement data 5 that is sent by a data source module 10 and transmitted 13 to the input interface 2 of the device 1.

The control module is configured, upon initiation of a respective next measurement cycle, to carry out a sequence of steps. The first step is to receive 13, via the input interface 2, respective next measurement data 5", 5‴ measured with respect to a patient 16. The second step is to process the respective next measurement data 5", 5"', based at least on a respective previous graph 6', 6" associated with a respective previous measurement cycle, for obtaining a respective next graph 6", 6‴ associated with the respective next measurement cycle. The third step is to output 15, via the output interface, the respective next graph 6", 6"'.

The previous graph 6', 6" and the next graph 6", 6‴ each relate to knowledge graphs 6, 9. These are illustrated by Fig. 3 and 4. As may be seen on these figures, they each comprise a plurality of entities relevant to a clinical scenario to which the respective measurement cycles relate, and a plurality of relations between the entities.

Fig. 1 thereby shows several instances of the next graphs that are updated. While not visible on the figure, the previous 6', 6" and the next graph 6", 6‴ are different; they differ in at least one entity or relation. This may be further understood from Fig. 4 (see below).

The previous measurement cycle and the next measurement cycle belong to a temporal sequence. The real-time measurements come in at a high refresh rate and hence do not constitute a bottleneck. The maximum time between respective cycles is kept below 10 seconds by fast computation of the next graph.

The device has an output interface 4 comprising electronic connection means for controlling a patient-related device 7, here a mechanical ventilator. The control module 3 is further configured to send 14, based on the difference between the graph associated with the previous measurement cycle and the graph associated with the new measurement cycle, an instruction to the mechanical ventilator 7 for altering the intensity of the flow of the ventilator.

The alteration of the ventilation flow is based on following measured data 5", 5"': arterial blood pressure, central venous pressure, a maximum pressure value of the mechanical ventilation cycle.

In variants of this example, the system as shown in Fig. 1 further includes (not shown) a peritoneal insufflator. The control module 3 is further configured to send, based on the difference between the graph associated with the previous measurement cycle and the graph associated with the new measurement cycle, an instruction to the peritoneal insufflator for altering the intensity of insufflation.

The alteration of the intensity of insufflation is based on following measurement data 5", 5‴: arterial blood pressure, central venous pressure, a pressure value characteristic of a mechanical ventilation cycle.

The device has an output interface 4 comprising electronic connection means for connecting to a user module comprising a screen 8 for display to an operator 17, and the control module 3 is further configured to display 15, via said screen 8, the next graph 6", 6‴.

The user module further comprises user input means for receiving input from the operator 17. Particularly, the screen 8 is a touchscreen, and thus acts both for display and for user input. The next graph 6', 6", 6‴ is displayed with a relation being editable by the doctor 17 via the touchscreen.

The control module 3 is further configured to determine, based at least on the next graph 6", 6"', whether an alert for the doctor 17 should be generated. If yes, the control module generates said alert and displays the alert via the touchscreen 8. In some cases, the alert is an instruction that may be carried out by the doctor 17 as part of the clinical procedure. In other cases, the alert is a prompt for the doctor 17. The doctor 17 thereby is informed of the predicted impact of their response, via an annotation of the displayed graph.

Fig. 1 further illustrates pre-determined patient data 19 specific to the patient 16, together with the real-time measurements constituting all patient data 18 that is available. This is the medical record that is available as offline resource (and thus, "received" 11 from the patient 16) and contains patient-specific information that is relevant to the clinical scenario. For instance, the pre-determined patient data 19 may indicate that insufflation should only be applied to a limited extent in view of the patient's medical history. Accordingly, a pre-determined patient-related graph may be instantiated based on pre-determined patient data 19 specific to the patient 16. This may relate to the graph illustrated by Fig. 3.

The data source module 10 is configured to measure next measurement data 5", 5‴ with respect to a patient 16, and send the next measurement data to the input interface 2 of the device.

Both the ventilator 7 (shown in Fig. 1) and the insufflator (not shown) may receive instructions from the device to alter an operational parameter. This may trigger the execution of an altering-related action. In this example, the altering-related action is (indeed) the altering of the operational parameter. In variants of this example (not further discussed), the action relates to generating a patient-related device alert, particularly an alarm sound, via an alerting means comprised in the patient-related device.

Fig. 2 illustrates the application of this system 100 to an environment with a patient 16 and a doctor 17. While the ventilator and insufflator are not shown separately in Fig. 2, they are both assumed to be present.

Turning to Fig. 3, the scenario-specific graph 9 relating to a first example clinical scenario that may involve both a respirator and an insufflator. It represents entities and their relations within this first example clinical scenario. Entities include, e.g., "Patient" (John Doe), "Procedure" (RALP), "Pathology" (COPD, Flow Limitation, Hypercarbia, Hypotension, RV Failure, Atelectasis), "Medical Device" (Mechanical Ventilator, Insufflator), and "Physiological Derangement." Relations (underlined) describe interactions between these entities. For example, John Doe suffers from COPD, which leads to Flow Limitation and, if synergistic with "MinVol" (minimal volume), increases IntraTx Pressure. Flow Limitation thereby synergistically acts along with MinVol to increase IntraTx pressure. On the other hand, MinVol reduces Hypercarbia. The RespFreq increases MinVol. The Insufflator provides Pneumoperitoneum, which increases Atelectasis, which in its turn increases Hypercarbia. RALP employs both the Mechanical Ventilator and the Insufflator. IntraTx pressure leads to RVFailure which leads to Hypotension. The graph thus captures cause-and-effect pathways, device interactions, and physiological impacts through directional relationships connecting the entities. The Physiological Derangement may thereby relate to an instantiation for a specific patient with, e.g., a flow limitation.

Regarding Fig. 3, for simplicity, not all instantiation relationships have been drawn, and the Pharmacology module is, while present, not shown.

On the other hand, Fig. 4 shows an example next graph 400 according to the invention, i.e., an intermediate graph that is recomputed for each next measurement cycle.

The graph is an example intermediate graph produced from semantic stream processing of the input data according to the invention. It is tailored to the context of a periodic acute hypotensive episode in a mechanically ventilated patient. For simplicity and illustrative purposes, the amount of graph nodes, node properties and inter-node relationships has been reduced significantly. The nodes "AIRWAY PRESSURE" and "ARTERIAL BP" (arterial blood pressure) represent a physiological concept for which an input stream serves as a direct or indirect (proxy) measure. In this example, Arterial BP could for example be obtained from the use of an intra-arterial catheter connected to a transducer. The airway pressure can be obtained from a sensor device that is part of a mechanical ventilator, or from any other commonly available measurement technique suitable to this purpose. The node "HYPOTENSION" is a detected feature that has been recognized as a clinical problem. The nodes "PEEP" and "Δ PRESSURE" represent concepts that have been designated as potential causes or contributing factors to the clinical problem. Other entities include "NORMOTENSION," "SIMV MODE" and "UNDERDAMPING," as well as timestamps ("16:03:50 UCT" and "08:01 :14 UCT"). Relations (underlined) connect these entities to indicate their temporal, causal, or observational relationships. For instance, "16:03:50 UCT" precedes "16:02:54 UCT" and onsets "Δ PRESSURE." "HYPOTENSION" is suspected to relate to "PEEP" and relates to "ARTERIAL BP" through a short term connection. "ARTERIAL BP" is further connected to "NORMOTENSION" over a medium term relation. Other key relationships include "AIRWAY PRESSURE," which has a short term relation to "Δ PRESSURE" and a long term relation to "SIMV MODE."

The graph of Fig. 4 thus visually represents causal and temporal interactions among various clinical measurements, conditions, and (time-stamped) events. As may be clear, each new event causes a new (time-stamped) entity to be created, and thus leads to the generation of a new graph. The next graph and the previous graph thereby differ with respect to this new event (new entity) and the associated relation(s) (new relation(s)). In variants of this example, the introduction of the new entity and new relation may thereby cause an entity and associated relations of the previous graph to be omitted.

(End of example)

## Claims

1. A device (1) comprising:
an input interface (2);
a control module (3) connected to the input interface;
an output interface (4) connected to said control module;
wherein the control module is configured, upon initiation of a respective next measurement cycle, to:
receive (13), via the input interface (2), respective next measurement data (5", 5‴) measured with respect to a patient (16);
process the respective next measurement data (5", 5‴), based at least on a respective previous graph (6', 6") associated with a respective previous measurement cycle, for obtaining a respective next graph (6", 6‴) associated with the respective next measurement cycle;
output (15), via the output interface, the respective next graph (6", 6‴);
wherein the previous graph (6', 6") and the next graph (6", 6‴) each relate to knowledge graphs (6, 9) and each comprise:
a plurality of entities relevant to a clinical scenario to which the respective measurement cycles relate, and
a plurality of relations between the entities; and
wherein the previous (6', 6") and the next graph (6", 6‴) differ in at least one entity or relation,
wherein **preferably** the previous measurement cycle and the next measurement cycle belong to a temporal sequence of at least five respective cycles, more preferably at least ten cycles, and wherein **preferably** a maximum time between respective cycles is less than one minute, more preferably less than 30 seconds, even more preferably less than 10 seconds.

2. The device (1) of claim 1, wherein the output interface (4) comprises electronic connection means for controlling a patient-related device (7), and wherein the control module (3) is further configured to:
send (14), based on the difference between the graph associated with the previous measurement cycle and the graph associated with the new measurement cycle, an instruction to the patient-related device (7) for altering an operational parameter of the patient-related device (7).

3. The device (1) of claim 2, wherein the patient-related device relates to a mechanical ventilator (7), wherein the operational parameter relates at least to an intensity of ventilation flow, and wherein the respective next measurement data (5", 5‴) relate to at least one of, preferably at least two of: arterial blood pressure, central venous pressure, a pressure value characteristic of a mechanical ventilation cycle.

4. The device (1) of claim 2 or 3, wherein the patient-related device (7) relates to a peritoneal insufflator, wherein the operational parameter relates at least to an intensity of insufflation, and wherein the respective next measurement data (5", 5‴) relate to at least one of, preferably at least two of: arterial blood pressure, central venous pressure, a pressure value characteristic of a mechanical ventilation cycle.

5. The device (1) of claims 1-4, wherein the output interface (4) comprises electronic connection means for connecting to a user module comprising a screen (8) for display to an operator (17), and wherein the control module (3) is further configured to:
display (15), via said screen (8), at least one graph (6', 6", 6‴), said at least one graph relating to at least one of the previous graph (6', 6") and the next graph (6", 6‴).

6. The device of claim 5, wherein the device comprises a postprocessing module relating to an LLM, with the at least one graph (6', 6", 6‴) as input prompt, whereby the LLM output is displayed to the operator (17), either along with the at least one graph (6', 6", 6‴), or instead of the at least one graph.

7. The device (1) of claim 6, wherein the user module further comprises user input means for receiving input from the operator (17), and wherein the at least one graph (6', 6", 6‴) being displayed comprises at least one entity and/or at least one relation that is editable by the operator (17) via said user input means.

8. The device (1) of claim 7, wherein the user input means relates to the screen being a touchscreen.

9. The device (1) of claims 6-8, wherein the control module (3) is further configured to:
determine, based at least on the next graph (6", 6‴), whether an alert for the operator (17) should be generated;
if yes, generate said alert and display the alert via said screen (8).

10. The device (1) of claim 9, wherein the alert relates to procedural information and comprises instructions to be carried out by the operator (17).

11. The device (1) of claims 9-10, wherein the user module further comprises user input means for receiving input from the operator (17), wherein the alert relates to a prompt for receiving a response from the operator (17) via said user input means, and wherein the control module (3) is further configured to:
display, on said screen and along with the prompt and the at least one displayed graph (6', 6", 6‴), an annotation of said displayed graph for indicating a predicted impact of the response.

12. The device (1) of claims 1-11, wherein the processing of the next measurement data (5ʺ, 5‴) for obtaining a respective next graph (6", 6‴) is further based on a pre-determined patient-related graph instantiated based on pre-determined patient data (19) specific to the patient (16).

13. A system (100) comprising:
a device (1), **preferably** the device (1) of any of claims 1-12, the device comprising
an input interface (2);
a control module (3) connected to the input interface;
an output interface (4) connected to said control module;
a data source module (10), preferably a sensor module, for connection to the input interface (2);
a user module comprising at least a screen (8) for display to an operator (17) and for connection to the output interface (4);
wherein the control module (3) is configured to:
receive (13), from the data source module (10), respective next measurement data (5", 5‴);
process the respective next measurement data (5", 5‴), based at least on a respective previous graph (6', 6") associated with a respective previous measurement cycle, for obtaining a respective next graph (6", 6‴) associated with the respective next measurement cycle;
send (15) data of the respective next graph (6", 6‴) to the user module;
wherein the data source module (10) is configured to:
measure next measurement data (5", 5‴) with respect to a patient (16), and
send the next measurement data to the input interface (2) of the device (1);
wherein the user module is configured to:
receive (15), from the output interface (4) of the device (1), data of at least one graph (6', 6", 6‴);
display, on said screen (8), said at least one graph (6', 6", 6‴);
wherein the previous graph (6', 6") and the next graph (6", 6‴) each relate to knowledge graphs (6, 9) and each comprise:
a plurality of entities relevant to a clinical scenario to which the respective measurement cycles relate, and
a plurality of relations between the entities; and
wherein the previous (6', 6") and the next graph (6", 6‴) differ in at least one entity or relation.

14. The system (100) of claim 13, further comprising:
a patient-related device (7) connected to the output interface (4);
wherein the control module (3) is further configured to:
send (14), based on the difference between the graph associated with the previous measurement cycle and the graph associated with the new measurement cycle, an instruction to the patient-related device (7) for altering an operational parameter of the patient-related device (7);
wherein the patient-related device (7) is configured to:
receive, from the output interface (4) of the device, the instruction for altering the operational parameter;
executing, based on said instruction, an altering-related action;
wherein the altering-related action relates to altering said operational parameter and/or generating a patient-related device alert via an alerting means comprised in the patient-related device, e.g., an acoustic alert.

15. Method for supporting a clinical event according to a clinical scenario, comprising the steps of:
receiving (13), via an input interface (2) comprised in a device (1), respective next measurement data (5", 5‴) measured with respect to a patient (16);
processing, by a control module (3) comprised in the device (1) the respective next measurement data (5", 5‴) based at least on a respective previous graph (6', 6") associated with a respective previous measurement cycle, for obtaining a respective next graph (6", 6‴) associated with the respective next measurement cycle;
outputting (15), via an output interface (4) comprised in the device, the respective next graph (6", 6‴);
wherein the previous graph (6', 6") and the next graph (6", 6‴) each relate to knowledge graphs (6, 9) and each comprise:
a plurality of entities relevant to the clinical scenario to which the respective measurement cycles relate, and
a plurality of relations between the entities; and
wherein the previous (6', 6") and the next graph (6", 6‴) differ in at least one entity or relation; wherein the method further comprises:
sending (14), based on the difference between the graph associated with the previous measurement cycle and the graph associated with the new measurement cycle, an instruction to the patient-related device (7) for altering an operational parameter of the patient-related device (7)
**and/or**
displaying (15), via a screen (8) comprised in a user module connected to the output interface (4) of the device (1), at least one graph (6', 6", 6‴), said at least one graph relating to at least one of the previous graph (6', 6") and the next graph (6", 6‴).
